# EUROPEAN PATENT SPECIFICATION

(11) **EP 2 515 641 B1**
(45) Date of publication and mention of the grant of the patent: **24.07.2013**
(21) Application number: 10822879.2
(22) Date of filing: 03.12.2010
(51) Int. Cl.: A01N 1/02, A61M 1/02

(54) **WRAPPING FOR CONTAINING AND PRESERVING A LIQUID SUBSTANCE INTENDED TO BE FROZEN**
VERPACKUNG ZUR AUFNAHME UND KONSERVIERUNG EINES EINZUFRIERENDEN FLÜSSIGEN STOFFES
EMBALLAGE DESTINÉ À CONTENIR ET À PRÉSERVER UNE SUBSTANCE LIQUIDE DESTINÉE À ÊTRE CONGELÉE

(30) Priority: 23.12.2009 IT MO20090311
(43) Date of publication of application: 31.10.2012
(73) Proprietor: Expertmed S.r.L., 37137 Verona (IT)
(72) Inventor: SGARBI, Massimo, I-37135 Verona (IT)
(74) Representative: Brunacci, Marco
(86) International application number: PCT/IB2010/003085
(87) International publication number: WO 2011/077206

(56) References cited:
- EP-A1- 0 608 121
- WO-A2-2004/110144
- WO-A2-2005/073652
- FR-A1- 2 845 352
- US-A- 4 117 881
- US-A1- 2005 180 998
- US-A1- 2007 032 774
- US-A1- 2007 084 222

## Description

### Technical Field

The present invention relates to a wrapping for containing and preserving a liquid substance intended to be frozen.

### Background Art

It is known that biological liquids, such as blood and its derivatives, are preserved inside special bags and undergo a freezing process so as to substantially maintain their properties unchanged over time.

More in particular, according to currently applicable laws, the required freezing must be such that the biological liquid inside the bag, in particular the liquid arranged in correspondence to the "heart" of the bag, has to reach a temperature of -30°C within a maximum time of one hour from when the bags are placed inside the relative freezing device.

After quickly freezing the bags, the latter are generally taken out and a label applied showing a series of data including the freezing data, the identification data of the liquid substance inside, the intended use, etc...

During the carrying out of this phase as well, during which the bags are positioned outside the freezer waiting to be stored in a freezer at a temperature of -40°C, it is crucial to monitor the pattern of the temperature of the liquid substance inside the bag.

Generally however, no direct control is made of these bag "processing" phases to monitor the biological liquid temperature pattern in a substantially continuous way.

This makes it very hard to establish a certification of the frozen bags in any way precise and above all complete with absolute parameters.

The patent application EP07000862 registered by the present applicant, describes a procedure for monitoring and certifying the freezing and unfreezing cycle of the bags containing a biological liquid.

Such procedure envisages the use of a synthetic liquid solution with freezing and unfreezing chemical-physical characteristics identical to those of the biological liquid that has to undergo the required heat treatment.

The procedure described by EP07000862 also envisages the reading and the recording of the temperatures achieved, starting from the time the freezing/unfreezing phase starts and according to a preset programme.

Such temperature data are then transferred to an electronic processor which analyses them and files them away.

EP07000862 does not however describe in which way, or according to which method, the temperature of the liquid solution can be read in the "heart" of the bag, i.e., in the area furthest from the external surfaces.

Some bags of known type used to store liquid substances are described by US 2005/180998, US 4117881, EP 0608121 and US 2007/084222.

More in particular, the bags described and represented in the above-mentioned documents are generally composed of two containment walls sealed together, the internal surfaces of which define the containment chamber of the liquid substance and the external surfaces of which are substantially smooth and define the external overall dimensions of the bag itself and have a seat for housing a reading device, e.g., suitable for reading the temperature of the liquid contained inside the bag itself.

The seats for housing the reading device described in the above documents are mainly communicating with the liquid containment chamber and correspond, e.g., to a duct for filling or draining the chamber itself.

This involves some drawbacks such as the possible contamination of the liquid contained in the bag due to direct contact with the reading device, or problems of leaking liquid, due to a not very good seal between the reading device and the containment walls delimiting the bag.

US 2005/180998 also describes an embodiment wherein the housing seat of the reading device is isolated from the liquid containment chamber and defined by a blind recess.

This embodiment also has a number of drawbacks however.

In fact, a bag or a container of this type does not allow the easy and safe positioning of the reading device. More in particular, the reading device housed inside the blind recess can be accidentally removed or moved from the relative seat following the movement of the bag, the movement of the liquid inside it or its stiffening due to the freezing of the liquid itself. These drawbacks therefore make the data read during the carrying out of the above monitoring and certification method unreliable, because the position of the temperature reading device can vary during the course of the freezing and unfreezing phase.

Furthermore, the bags and the containers of this type are complicated to make and therefore have a high cost.

### Description of the Invention

The aim of the present invention is to provide a wrapping for containing and preserving a liquid solution, particularly a biological liquid, which allows easy and safe fitting/removal of a temperature reading device.

In particular, the present invention proposes allowing the stable positioning of the reading device so that the read data are as reliable as possible.

Within such aim, one object of the present invention is to provide a wrapping for containing and preserving liquid substances that allows reading the temperature of the liquid substance contained in the heart of the bag, i.e. in correspondence to the area furthest from the external surfaces.

One object of the present invention therefore is to provide a wrapping for containing and preserving liquid substances that allows safely and reliably monitoring the temperature of the liquid substance contained inside during one or more of the above-mentioned processing phases, i.e., during the phase of freezing, subsequent labelling, storage and unfreezing.

Yet another object is to provide a wrapping which is effective and safe to use besides being easy to make and not too expensive.

Another object of the present invention is to provide a wrapping for containing and preserving a liquid substance, in particular a biological liquid, which allows overcoming the mentioned drawbacks of the state of the art within the ambit of a simple, rational, easy and effective to use as well as low cost solution. The above objects are achieved by the present wrapping for containing and preserving a liquid substance intended to be frozen, comprising at least two containment walls associated the one with the other to define a chamber for containing the liquid substance and at least a seat, isolated from said containment chamber, for housing a temperature reading device for reading the temperature of the liquid substance, where said containment walls comprise at least two outer walls which substantially define the outer overall dimensions of the wrapping itself and at least one supporting wall which delimits at least said seat and which is intended to support said temperature reading device, characterised by the fact that said at least one supporting wall is configured so as to define a through opening through said containment chamber and delimiting said seat.

### Brief Description of the Drawings

Other characteristics and advantages of the present invention will become more evident from the description of a preferred, but not sole, embodiment of a wrapping for containing and preserving a liquid substance intended to be frozen, particularly a biological liquid, illustrated purely as an example but not limited to the annexed drawings in which:

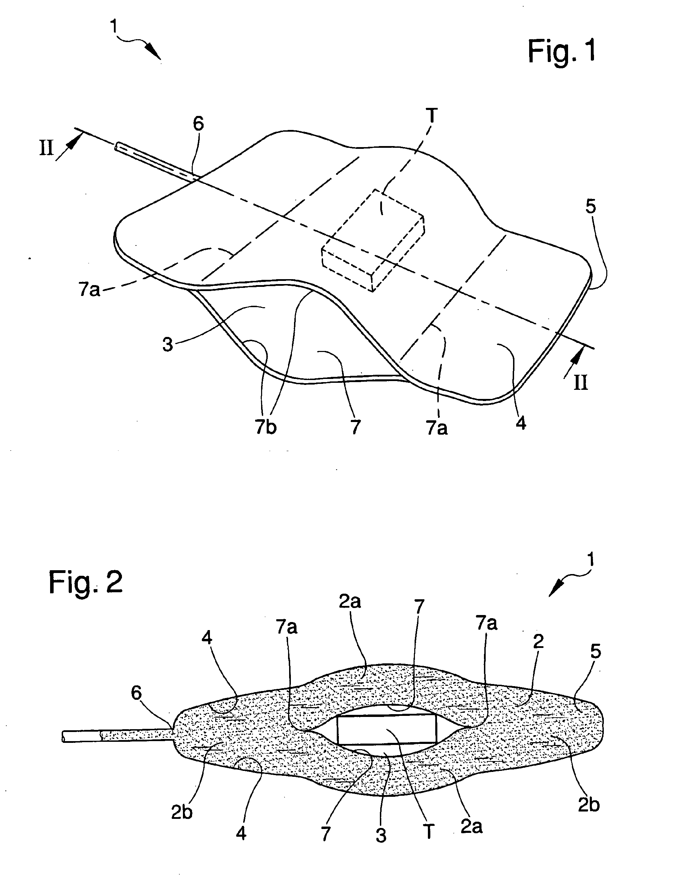

figure 1 is an axonometric view of a first embodiment of a wrapping according to the invention;
figure 2 is a section view of the wrapping of figure 1 along a track plane II-II.

### Embodiments of the Invention

With particular reference to such figures, globally indicated by reference number 1 is a wrapping for containing and preserving a liquid substance S intended to be frozen.

The wrapping 1 comprises at least two containment walls 4,7 associated the one with the other to define at least a chamber 2 for containing the liquid substance S. More in particular, the liquid substance S can be a synthetic substance having chemical-physical freezing and unfreezing characteristics similar to those of a biological liquid of reference which has to undergo the desired heat treatment or can be the biological liquid itself such as, e.g., blood and its derivatives. In the case of the liquid substance S being of the type of a biological liquid, the containment walls 4,7 are made using biocompatible materials of the type known to the expert in the sector, such as those generally used for bags for containing blood and plasma, i.e., PVC, polyurethane, EVA and the like.

The wrapping 1, and more in particular, the containment walls 4,7, can be both of the flexible type or of the rigid type.

In the embodiments shown in the illustrations, the wrapping 1 is of the flexible type and the containment walls 4,7 comprise at least two outer walls 4, which delimit at least partially the chamber 2 and which substantially define the outside overall dimensions of the wrapping itself.

More in particular, the outer walls 4 externally delimit the chamber 2 for containing the liquid substance S and are associated with one another along at least a part of their peripheral contour, e.g., by sealing, in such a way as to form a perimeter edge 5 which defines a closed or closable profile. The perimeter edge 5 does in fact generally have at least an inlet gap 6 for the liquid substance S suitable for being subsequently closed.

The wrapping 1 then comprises at least a seat 3, isolated from the chamber 2, for housing a temperature reading device T of the liquid substance S. The containment walls 4,7 comprise at least a supporting wall 7 suitable for delimiting the seat 3.

The device T is therefore intended to be arranged outside the chamber itself, without therefore being in contact with the substance contained in the same. According to the invention, the supporting wall 7 is configured so as to define a through opening through the chamber 2 and delimiting the seat 3.

With its internal surface, the supporting wall 7 delimits the chamber 2, and with its external surface, the seat 3 which houses the temperature reading device T. Suitably, the supporting wall 7 extends from at least one of the outer walls 4. Advantageously, the seat 3 is accessible from the outside so that an operator can easily fit and remove the device T from the seat itself.

Preferably, the seat 3 is arranged in correspondence to the median area of the containment chamber 2, so that the device T is able to read the temperature of the liquid substance S in correspondence to the central area of the chamber itself. The seat 3 is therefore arranged so that the device T is placed in correspondence to the central area of the chamber 2 and away from the outer walls 4 which delimit it.

More in particular, the seat 3, and therefore the supporting wall 7 which delimits it, is placed between the outer walls 4.

The device T used to read and monitor the temperature generally comprises a temperature sensor connected to a processor or data logger suitable for receiving and processing the signals arriving from the temperature sensor itself. The temperature sensor can be made in a single piece with the processor, as shown in the illustrations, or be connected to this by cable or by means of data transmission means of known type.

More in detail, in the preferred but not only embodiment shown in the illustrations, the through opening 3 extends between two substantially opposite sections of the perimeter edge 5.

In this first embodiment, the through opening 3 is delimited by at least two supporting walls 7 associated the one with the other and associated with a respective outer wall 4.

Such supporting walls 7 which delimit the through opening 3 stand out from the outer walls 4 and have a substantially quadrilateral conformation, i.e., they have two first sides 7a opposite one another and two second sides 7b opposite one another and adjacent to the respective first sides 7a. More in detail, the supporting walls 7 are associated with one another by means of the respective first sides 7a and each of them is associated with the corresponding outer wall 4 by means of its second sides 7b.

As has already been said above, in this first embodiment, the seat 3 is therefore placed between the outer walls 4 and the chamber 2 for containing the liquid substance S comprises two containment areas 2a which are distinct from one another and are separated from the supporting walls 7 and two intermediate areas 2b arranged on opposite sides of the supporting walls 7 and communicating with both the areas 2a.

The device T positioned inside the seat 3 is therefore substantially surrounded by the containment areas 2a and by the intermediate areas 2b, and is therefore substantially immersed in the liquid substance S.

The expert in the sector can immediately understand how the device T is fitted and removed from the seat 3.

It has in actual facts been ascertained how the described invention achieves the proposed objects and in particular the fact is underlined that the wrapping according to the invention allows applying a temperature reading device in a safe and easy way.

More in particular, the fact that the seat for housing the temperature reading device is defined by a through opening allows avoiding that such device move during the freezing/unfreezing phases, thus facilitating more accurate and reliable reading compared to the use of the wrappings of known type. Furthermore, the wrapping according to the invention allows reading, in a precise and easy way, the temperature of the liquid substance in correspondence to the "heart" of the relative containment chamber.

Further still, the wrapping according to the invention allows performing in a reliable way a method for monitoring the freezing and unfreezing phase of the liquid substance, such as that described in EP07000862. The wrapping according to the invention is also simple to make.

## Claims

1. Wrapping (1) for containing and preserving a liquid substance (S) intended to be frozen, comprising at least two containment walls (4, 7) associated the one with the other to define a chamber (2) for containing the liquid substance (S) and at least a seat (3), isolated from said containment chamber (2), for housing a temperature reading device (T) for reading the temperature of the liquid substance (S), where said containment walls (4, 7) comprise at least two outer walls (4) which substantially define the outer overall dimensions of the wrapping itself and at least one supporting wall (7) which delimits at least said seat (3) and which is intended to support said temperature reading device (T), **characterised by** the fact that said at least one supporting wall (7) is configured so as to define a through opening through said containment chamber (2) and delimiting said seat (3).

2. Wrapping (1) according to claim 1, **characterised by** the fact that said through opening (3) is delimited by at least two of said supporting walls (7) placed between said outer walls (4) and associated the one with the other, each of said supporting walls (7) also being associated with one of said outer walls (4) in such a way that said containment chamber (2) has two containment areas (2a) communicating with each other and separated from the supporting walls themselves.

3. Wrapping (1) according to claim 2, **characterised by** the fact that each of said supporting walls (7) comprises a pair of first sides (7a) opposite one another and a pair of second sides (7b) opposite one another and adjacent to said first sides (7a), said supporting walls (7) being associated with one another by means of the respective first sides (7a) and being associated with a respective outer wall (4) by means of said second sides (7b).

4. Wrapping (1) according to one or more of the preceding claims, **characterised by** the fact that said housing seat (3) is defined in correspondence to a median area of said containment chamber (2).

5. Wrapping (1) according to one or more of the preceding claims, **characterised by** the fact that said seat (3) is accessible from the outside.

6. Wrapping (1) according to one or more of the preceding claims, **characterised by** the fact that said at least one supporting wall (7) extends from at least one of said outer walls (4).

7. Wrapping (1) according to one or more of the preceding claims, **characterised by** the fact that said seat (3) is substantially placed between said outer walls (4).

8. Wrapping (1) according to one or more of the preceding claims, **characterised by** the fact that said outer walls (4) are associated the one with the other so as to define a perimeter edge (5) of the wrapping itself and **characterised by** the fact that said through opening (3) extends between two substantially opposite sections of said perimeter edge (5).

## Patentansprüche

1. Verpackung (1) zur Aufnahme und Konservierung eines zum Einfrieren vorgesehenen flüssigen Stoffes (S), enthaltend mindestens zwei Behälterwände (4, 7), die miteinander verbunden sind, so dass sie eine Kammer (2) zur Aufnahme des flüssigen Stoffes (S) bilden, und mindestens einen Sitz (3), der von der Behälterkammer (2) isoliert ist, zur Unterbringung einer Temperaturmesseinrichtung (T) zum Messen der Temperatur des flüssigen Stoffes (S), wobei die Behälterwände (4, 7) mindestens zwei Außenwände (4) aufweisen, welche im Wesentlichen die äußeren Gesamtabmessungen der Verpackung selbst bestimmen, und mindestens eine Stützwand (7), welche mindestens den Sitz (3) begrenzt und welche dafür vorgesehen ist, die Temperaturmesseinrichtung (T) zu tragen, **gekennzeichnet durch** die Tatsache, dass die mindestens eine Stützwand (7) so konfiguriert ist, dass sie eine Durchgangsöffnung durch die Behälterkammer (2) bildet und den Sitz (3) begrenzt.

2. Verpackung (1) nach Anspruch 1, **gekennzeichnet durch** die Tatsache, dass die Durchgangsöffnung (3) von mindestens zwei Stützwänden (7) begrenzt ist, die zwischen die Außenwände (4) gesetzt sind und miteinander verbunden sind, wobei jede der Stützwände (7) auch mit einer der Außenwände (4) dergestalt verbunden ist, dass die Behälterkammer (2) zwei Behälterbereiche (2a) aufweist, die miteinander in Verbindung stehen und von den Stützwänden selbst getrennt sind.

3. Verpackung (1) nach Anspruch 2, **gekennzeichnet durch** die Tatsache, dass jede der Stützwände (7) ein Paar erste Seiten (7a), die einander gegenüberliegen, und ein Paar zweite Seiten (7b) aufweist, die einander gegenüberliegen und an die ersten Seiten (7a) angrenzen, wobei die Stützwände (7) mittels der jeweiligen ersten Seiten (7a) miteinander verbunden sind und mit einer jeweiligen Außenwand (4) mittels der zweiten Seiten (7b) verbunden sind.

4. Verpackung (1) nach einem oder mehreren der vorhergehenden Ansprüche, **gekennzeichnet durch** die Tatsache, dass der Unterbringungssitz (3) in Übereinstimmung mit einem Mittelbereich der Behälterkammer (2) gebildet ist.

5. Verpackung (1) nach einem oder mehreren der vorhergehenden Ansprüche, **gekennzeichnet durch** die Tatsache, dass der Sitz (3) von außen zugänglich ist.

6. Verpackung (1) nach einem oder mehreren der vorhergehenden Ansprüche, **gekennzeichnet durch** die Tatsache, dass die mindestens eine Stützwand (7) von mindestens einer der Außenwände (7) ausgeht.

7. Verpackung (1) nach einem oder mehreren der vorhergehenden Ansprüche, **gekennzeichnet durch** die Tatsache, dass der Sitz (3) im Wesentlichen zwischen den Außenwänden (4) platziert ist.

8. Verpackung (1) nach einem oder mehreren der vorhergehenden Ansprüche, **gekennzeichnet durch** die Tatsache, dass die Außenwände (4) miteinander verbunden sind, so dass sie einen Umfangsrand (5) der Verpackung selbst bilden, und **gekennzeichnet durch** die Tatsache, dass die Durchgangsöffnung (3) zwischen zwei im Wesentlichen entgegengesetzten Abschnitten des Umfangsrandes (5) verläuft.

## Revendications

1. Emballage (1) pour contenir et conserver une substance liquide (S) destinée à être congelée, comprenant au moins deux parois de confinement (4, 7) associées l'une à l'autre pour définir une chambre (2) prévue pour contenir la substance liquide (S) et au moins un compartiment (3), isolé de ladite chambre de confinement (2), prévu pour loger un dispositif de lecture de température (T) destiné à la lecture de la température de la substance liquide (S), lesdites parois de confinement (4, 7) comprenant au moins deux parois externes (4) qui définissent sensiblement les dimensions globales externes de l'emballage lui-même et au moins une paroi de support (7) qui délimite au moins ledit compartiment (3) et qui est destinée à porter ledit dispositif de lecture de température (T), **caractérisé par le fait que** ladite au moins une paroi de support (7) est configurée de manière à définir une ouverture transversale à travers ladite chambre de confinement (2) et à délimiter ledit compartiment (3).

2. Emballage (1) selon la revendication 1, **caractérisé par le fait que** ladite ouverture transversale (3) est délimitée par au moins deux desdites parois de support (7) placées entre lesdites parois externes (4) et associées l'une à l'autre, chacune desdites parois de support (7) étant également associée à l'une desdites parois externes (4) d'une manière telle que ladite chambre de confinement (2) comporte deux zones de confinement (2a) communiquant l'une avec l'autre et séparées des parois de support elles-mêmes.

3. Emballage (1) selon la revendication 2, **caractérisé par le fait que** chacune desdites parois de support (7) comprend une paire de premiers bords (7a) opposés l'un à l'autre et une paire de deuxièmes bords (7b) opposés l'un à l'autre et adjacents auxdits premiers bords (7a), lesdites parois de support (7) étant associées l'une à l'autre au moyen des premiers bords (7a) respectifs et étant associées à une paroi externe (4) respective au moyen desdits deuxièmes bords (7b).

4. Emballage (1) selon une ou plusieurs des revendications précédentes, **caractérisé par le fait que** ledit compartiment de logement (3) est défini en correspondance avec une zone médiane de ladite chambre de confinement (2).

5. Emballage (1) selon une ou plusieurs des revendications précédentes, **caractérisé par le fait que** ledit compartiment (3) est accessible de l'extérieur.

6. Emballage (1) selon une ou plusieurs des revendications précédentes, **caractérisé par le fait que** ladite au moins une paroi de support (7) s'étend à partir d'au moins l'une desdites parois externes (4).

7. Emballage (1) selon une ou plusieurs des revendications précédentes, **caractérisé par le fait que** ledit compartiment (3) est placé sensiblement entre lesdites parois externes (4).

8. Emballage (1) selon une ou plusieurs des revendications précédentes, **caractérisé par le fait que** lesdites parois externes (4) sont associées l'une à l'autre de manière à définir une bordure (5) formant un périmètre de l'emballage lui-même et **caractérisé par le fait que** ladite ouverture transversale (3) s'étend entre deux parties sensiblement opposées de ladite bordure (5) formant un périmètre.
